(19) Europäisches Patentamt
European Patent Office
Office européen des brevets

(11) Veröffentlichungsnummer: **0 008 091**
**B1**

(12) # EUROPÄISCHE PATENTSCHRIFT

(45) Veröffentlichungstag der Patentschrift: 12.08.81

(51) Int. Cl.³: **C 07 D 231/12**, C 07 D 231/16, A 01 N 43/56 // C07C103/365

(21) Anmeldenummer: **79102794.9**

(22) Anmeldetag: **03.08.79**

(54) **Substituierte N-Pyrazolylmethyl-halogenacetanilide, Verfahren zu ihrer Herstellung sowie ihre Verwendung als Herbizide.**

(30) Priorität: **10.08.78 DE 2835156**

(43) Veröffentlichungstag der Anmeldung: **20.02.80 Patentblatt 80/4**

(45) Bekanntmachung des Hinweises auf die Patenterteilung: **12.08.81 Patentblatt 81/32**

(84) Benannte Vertragsstaaten: **AT BE CH DE FR GB IT NL SE**

(56) Entgegenhaltungen:
**DE-A-2 704 281**
**DE-A-2 742 583**
**FR-A-2 368 476**

(73) Patentinhaber: **BAYER AG, Zentralbereich Patente, Marken und Lizenzen, D-5090 Leverkusen 1, Bayerwerk (DE)**

(72) Erfinder: **Thomas, Rudolf, Dr., Wilkhausstrasse 129, D-5600 Wuppertal 2 (DE)**
Erfinder: **Schmidt, Thomas, Dr., Am Bandenfeld 72, D-5657 Haan (DE)**
Erfinder: **Stetter, Jörg, Dr., Pahlkestrasse 3, D-5600 Wuppertal 1 (DE)**
Erfinder: **Eue, Ludwig, Dr., Paul-Klee-Strasse 36, D-5090 Leverkusen (DE)**
Erfinder: **Schmidt, Robert Rudolf, Dr., Hahnenweg 5, D-5000 Köln 80 (DE)**

**EP 0 008 091 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Substituierte N-Pyrazolylmethyl-halogenacetanilide, Verfahren zu ihrer Herstellung sowie ihre Verwendung als Herbizide

Die vorliegende Erfindung betrifft neue substituierte N-Pyrazolylmethyl-halogenacetanilide, ein Verfahren zu ihrer Herstellung sowie ihre Verwendung als Herbizide.

Es ist bereits bekannt geworden, daß man 2,6-Diethyl-N-methoxymethyl-chloracetanilid zur selektiven Unkrautbekämpfung verwenden kann (vgl. R. Wegler, Chemie der Pflanzenschutz- und Schädlingsbekämpfungsmittel, Band 5, Seite 255, Springer-Verlag (1977)). Diese Verbindung ist jedoch nicht immer ausreichend wirksam und in ihrer Selektivität nicht immer ganz befriedigend.

Weiterhin ist bereits bekannt geworden, daß zahlreiche N-substituierte Halogenacetanilide, in denen das Stickstoffatom des Anilinteiles über eine $CH_2$-Gruppe mit einem gegebenenfalls substituierten Azol über ein N-Atom des Azol-Restes verbunden ist, gute herbizide Eigenschaften besitzen (vergleiche FR-A 2 368 476, DE-A 2 704 281 und DE-A 2 742 583). Die selektive herbizide Wirksamkeit dieser Stoffe läßt jedoch in manchen Fällen zu wünschen übrig.

Es wurden nun als neue Verbindungen die substituierten N-Pyrazolylmethyl-halogenacetanilide der Formel

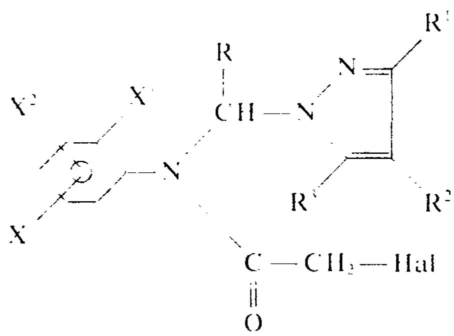

(I)

in welcher

Hal fur Fluor, Chlor oder Brom steht,

R für Alkyl mit 1 bis 8 Kohlenstoffatomen, Cycloalkyl mit 3 bis 6 Kohlenstoffatomen, Halogenalkyl mit bis zu 2 Kohlenstoffatomen und bis zu 3 Halogenatomen, Alkoxyalkyl mit 1 bis 4 Kohlenstoffatomen im Alkylteil und 1 bis 4 Kohlenstoffatomen im Alkoxyteil, für Alkenyl mit 2 bis 4 Kohlenstoffatomen, Alkinyl mit 2 bis 4 Kohlenstoffatomen oder für gegebenenfalls durch Halogen, Alkyl mit 1 bis 4 Kohlenstoffatomen, Halogenalkyl mit bis zu 2 Kohlenstoffatomen und bis zu 3 Halogenatomen, Alkoxy mit bis zu 2 Kohlenstoffatomen, Alkylthio mit bis zu 2 Kohlenstoffatomen, Cyano und/oder Nitro substituiertes Phenyl steht,

$R^1$, $R^2$ und $R^3$ gleich oder verschieden sind und für Wasserstoff, Alkyl mit 1 bis 4 Kohlenstoffatomen, Halogen oder Alkoxy mit 1 bis 4 Kohlenstoffatomen stehen und

$X^1$, $X^2$ und $X^3$ gleich oder verschieden sind und für Wasserstoff oder Alkyl mit 1 bis 4 Kohlenstoffatomen stehen,

und deren physiologisch verträgliche Säureadditions-Salze und Metallsalz-Komplexe gefunden.

Weiterhin wurde gefunden, daß man die substituierten N-Pyrazolylmethyl-halogenacetanilide der Formel (I) erhält, wenn man Halogenacetanilide der Formel

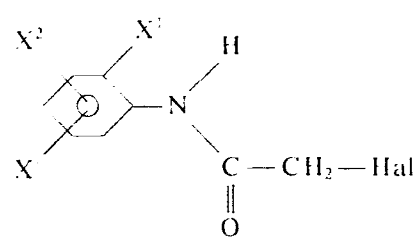

(II)

in welcher

Hal, $X^1$, $X^2$ und $X^3$ die oben angegebene Bedeutung haben,

mit Pyrazolyl-Derivaten der Formel

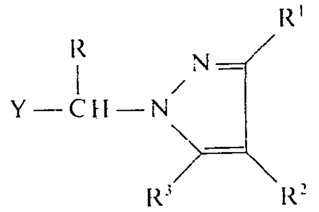

(III)

in welcher
R, $R^1$, $R^2$ und $R^3$ die oben angegebene Bedeutung haben und
Y für Halogen, den Mesyl- oder Tosyl-Rest steht,
in Gegenwart eines Säurebinders und gegebenenfalls in Gegenwart eines organischen Lösungsmittels umsetzt und gegebenenfalls anschließend eine Säure oder ein Metallsalz, addiert, wobei die Pyrazol-Derivate der Formel (III) vorzugsweise in Form von Halogenwasserstoffsalzen eingesetzt werden.

Außerdem wurde gefunden, daß die substituierten N-Pyrazolyl-methyl-halogenacetanilide der Formel (I) starke herbizide, insbesondere auch selektiv-herbizide Eigenschaften aufweisen.

Überraschenderweise zeigen die erfindungsgemäßen substituierten N-Pyrazolylmethyl-halogenacetanilide gegenüber dem bekannten 2,6-Diethyl-N-methoxymethyl-chloracetanilid bei gleichwertiger Unkrautwirkung bessere Möglichkeiten zum Einsatz als selektive Unkrautbekämpfungsmittel.

Ferner übertreffen die erfindungsgemäßen substituierten N-Pyrazolylmethyl-halogenacetanilide auch konstituionell ähnliche Stoffe, die in der FR-A 2 368 476 bzw. in der DE-A 2 704 281 offenbart werden, bezüglich ihrer selektiven herbiziden Wirksamkeit. So läßt sich das erfindungsgemäße 2,6-Dimethyl-N-(pyrazol-1-yl-eth-1-yl)-chloracetanilid besser zur selektiven Unkrautbekämpfung in Sinapis einsetzen als das 2,6-Diethyl-N-(pyrazol-1-yl-methyl)-chloracetanilid, das sowohl in der FR-A 2 368 476 als auch in der DE-A 2 704 281 beschrieben wird. Darüberhinaus besitzen das 2-Ethyl-6-methyl-N-(pyrazol-1-yl-eth-1-yl)-chloracetanilid und das 2,6-Dimethyl-N-(pyrazol-1-yl-eth-1-yl)-chloracetanilid überraschenderweise bei der Unkrautbekämpfung in Weizen, Baumwollen, Mais und Rüben bessere selektive herbizide Eigenschaften als das vorbekannte 2,6-Dietnyl-N-(1,2,4-triazol-1-yl-methyl)-chloracetanilid.

Die erfindungsgemäßen Stoffe stellen somit eine wertvolle Bereicherung der herbiziden Mittel zur selektiven Unkrautbekämpfung dar.

Die unsymmetrisch substituierten N-Pyrazolyl-methylhalogenacetanilide der Formel (I) treten aufgrund tautomerer Strukturen in den zur Herstellung verwendeten Ausgangsstoffen in zwei isomeren Formen auf, die sich formelmäßig wie folgt veranschaulichen lassen:

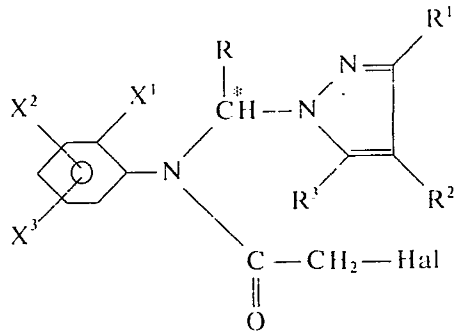

(a)

und

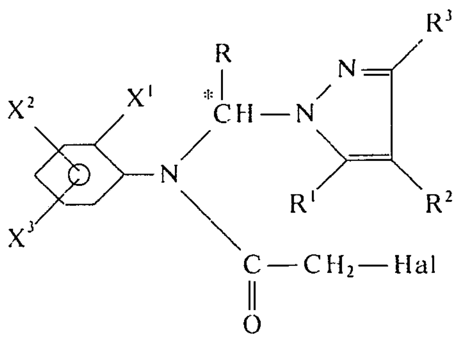

(b)

Das Isomeren-Verhältnis wird im wesentlichen von der Art der Pyrazol-Substituenten bestimmt. Außerdem können die Verbindungen der Formel (I) als optische Isomere vorliegen (vgl. durch x markiertes Kohlenstoffatom). Meist fallen jedoch Gemische an, die alle Isomeren enthalten. – Die Formel (I) umfaßt sowohl die Stellungsisomeren als auch die optischen Isomeren.

Ganz besonders bevorzugt sind diejenigen Verbindungen der Formel (I), in denen Hal für Chlor oder Brom steht; R für Methyl, Ethyl, n-Propyl, Isopropyl, Isobutyl, sek.-Butyl, Cyclopropyl, Cyclopentyl, Cyclohexyl, Trifluormethyl, Chlormethyl, Dichlormethyl, Trichlormethyl, Tribrommethyl, Vinyl, Allyl, Propen-1-yl, Ethinyl, Propargyl, Phenyl, Chlorphenyl, Dichlorphenyl, Methylphenyl, Dimethylphenyl, Chlormethylphenyl oder Nitrophenyl steht; $R^1$, $R^2$ und $R^3$ gleich oder verschieden sind und für Wasserstoff, Methyl, Chlor, Brom oder Methoxy stehen; und $X^1$, $X^2$ und $X^3$ gleich oder verschieden sind und für Wasserstoff, Methyl, Ethyl, n-Propyl, Isopropyl, Isobutyl, sek.-Butyl oder tert.-Butyl stehen.

Als Beispiele für erfindungsgemäße substituierte N-Pyrazol-yl-methyl-halogenacetanilide der Formel (I) seien im einzelnen genannt:

Tabelle 1

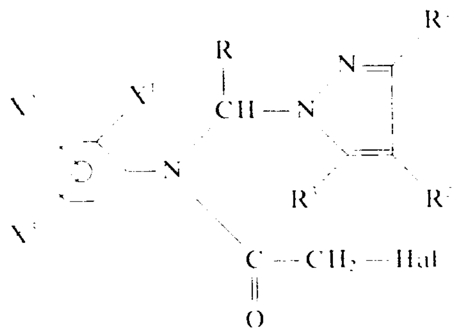

(I)

| $X^1$ | $X^2$ | $X^3$ | R | $R^1$ | $R^2$ | $R^3$ | Hal |
|---|---|---|---|---|---|---|---|
| H | H | H | $CH_3$ | H | H | H | Cl(Br) |
| H | H | H | $CH_3$ | H | Cl | H | Cl(Br) |
| H | H | H | $CH_3$ | $CH_3$ | H | $CH_3$ | Cl(Br) |
| H | H | H | $CH_3$ | $CH_3$ | H | H | Cl(Br) |
| H | H | H | $CH_3$ | H | H | CH | Cl(Br) |
| H | H | H | $CH_3$ | $CH_3$ | Cl | $CH_3$ | Cl(Br) |
| H | H | H | $CH_3$ | H | $OCH_3$ | H | Cl(Br) |
| 2-$CH_3$ | H | H | $CH_3$ | H | H | H | Cl(Br) |
| 2-$CH_3$ | H | H | $CH_3$ | H | Cl | H | Cl(Br) |
| 2-$CH_3$ | H | H | $CH_3$ | $CH_3$ | H | $CH_3$ | Cl(Br) |
| 2-$CH_3$ | H | H | $CH_3$ | $CH_3$ | H | H | Cl(Br) |
| 2-$CH_3$ | H | H | $CH_3$ | H | H | CH | Cl(Br) |
| 2-$CH_3$ | H | H | $CH_3$ | $CH_3$ | Cl | CH | Cl(Br) |
| 2-$CH_3$ | H | H | $CH_3$ | H | $OCH_3$ | H | Cl(Br) |
| 2-$C_2H_5$ | H | H | $CH_3$ | H | H | H | Cl(Br) |
| 2-$C_2H_5$ | H | H | $CH_3$ | H | Cl | H | Cl(Br) |
| 2-$C_2H_5$ | H | H | $CH_3$ | $CH_3$ | H | CH | Cl(Br) |

Fortsetzung

| $X^1$ | $X^2$ | $X^3$ | R | $R^1$ | $R^2$ | $R^3$ | Hal |
|---|---|---|---|---|---|---|---|
| 2-$C_2H_5$ | H | H | $CH_3$ | $CH_3$ | H | H | Cl(Br) |
| 2-$C_2H_5$ | H | H | $CH_3$ | H | H | $CH_3$ | Cl(Br) |
| 2-$C_2H_5$ | H | H | $CH_3$ | $CH_3$ | Cl | $CH_3$ | Cl(br) |
| 2-$C_2H_5$ | H | H | $CH_3$ | H | $OCH_3$ | H | Cl(Br) |
| 2-i-$C_3H_7$ | H | H | $CH_3$ | H | H | H | Cl(Br) |
| 2-i-$C_3H_7$ | H | H | $CH_3$ | H | Cl | H | Cl(Br) |
| 2-i-$C_3H_7$ | H | H | $CH_3$ | $CH_3$ | H | $CH_3$ | Cl(Br) |
| 2-i-$C_3H_7$ | H | H | $CH_3$ | $CH_3$ | H | H | Cl(Br) |
| 2-i-$C_3H_7$ | H | H | $CH_3$ | H | H | $CH_3$ | Cl(Br) |
| 2-i-$C_3H_7$ | H | H | $CH_3$ | $CH_3$ | Cl | $CH_3$ | Cl(Br) |
| 2-i-$C_3H_7$ | H | H | $CH_3$ | H | $OCH_3$ | H | Cl(Br) |
| 2-sek.-$C_4H_9$ | H | H | $CH_3$ | H | H | H | Cl(Br) |
| 2-sek.-$C_4H_9$ | H | H | $CH_3$ | H | Cl | H | Cl(Br) |
| 2-sek.-$C_4H_9$ | H | H | $CH_3$ | $CH_3$ | H | $CH_3$ | Cl(Br) |
| 2-sek.-$C_4H_9$ | H | H | $CH_3$ | $CH_3$ | H | H | Cl(Br) |
| 2-sek.-$C_4H_9$ | H | H | $CH_3$ | H | H | $CH_3$ | Cl(Br) |
| 2-sek.-$C_4H_9$ | H | H | $CH_3$ | $CH_3$ | Cl | $CH_3$ | Cl(Br) |
| 2-sek.-$C_4H_9$ | H | H | $CH_3$ | H | $OCH_3$ | H | Cl(Br) |
| 2-tert.-$C_4H_9$ | H | H | $CH_3$ | H | H | H | Cl(Br) |
| 2-tert.-$C_4H_9$ | H | H | $CH_3$ | H | Cl | H | Cl(Br) |
| 2-tert.-$C_4H_9$ | H | H | $CH_3$ | $CH_3$ | H | $CH_3$ | Cl(Br) |
| 2-tert.-$C_4H_9$ | H | H | $CH_3$ | $CH_3$ | H | H | Cl(Br) |
| 2-tert.-$C_4H_9$ | H | H | $CH_3$ | H | H | $CH_3$ | Cl(Br) |
| 2-tert.-$C_4H_9$ | H | H | $CH_3$ | $CH_3$ | Cl | $CH_3$ | Cl(Br) |
| 2-tert.-$C_4H_9$ | H | H | $CH_3$ | H | $OCH_3$ | H | Cl(Br) |
| 2-$CH_3$ | 6-$CH_3$ | H | $CH_3$ | H | H | H | Cl(Br) |
| 2-$CH_3$ | 6-$CH_3$ | H | $CH_3$ | H | Cl | H | Cl(Br) |
| 2-$CH_3$ | 6-$CH_3$ | H | $CH_3$ | $CH_3$ | H | $CH_3$ | Cl(Br) |
| 2-$CH_3$ | 6-$CH_3$ | H | $CH_3$ | $CH_3$ | H | H | Cl(Br) |
| 2-$CH_3$ | 6-$CH_3$ | H | $CH_3$ | H | H | $CH_3$ | Cl(Br) |
| 2-$CH_3$ | 6-$CH_3$ | H | $CH_3$ | $CH_3$ | Cl | $CH_3$ | Cl(Br) |

Fortsetzung

| $X^1$ | $X^2$ | $X^3$ | R | $R^1$ | $R^2$ | $R^3$ | Hal |
|---|---|---|---|---|---|---|---|
| 2-$CH_3$ | 6-$CH_3$ | H | $CH_3$ | H | $OCH_3$ | H | Cl(Br) |
| 2-$C_2H_5$ | 6-$CH_3$ | H | $CH_3$ | H | H | H | Cl(Br) |
| 2-$C_2H_5$ | 6-$CH_3$ | H | $CH_3$ | H | Cl | H | Cl(Br) |
| 2-$C_2H_5$ | 6-$CH_3$ | H | $CH_3$ | $CH_3$ | H | $CH_3$ | Cl(Br) |
| 2-$C_2H_5$ | 6-$CH_3$ | H | $CH_3$ | $CH_3$ | H | H | Cl(Br) |
| 2-$C_2H_5$ | 6-$CH_3$ | H | $CH_3$ | H | H | $CH_3$ | Cl(Br) |
| 2-$C_2H_5$ | 6-$CH_3$ | H | $CH_3$ | $CH_3$ | Cl | $CH_3$ | Cl(Br) |
| 2-$C_2H_5$ | 6-$CH_3$ | H | $CH_3$ | H | $OCH_3$ | H | Cl(Br) |
| 2-$C_2H_5$ | 6-$C_2H_5$ | H | $CH_3$ | H | H | H | Cl(Br) |
| 2-$C_2H_5$ | 6-$C_2H_5$ | H | $CH_3$ | H | Cl | H | Cl(Br) |
| 2-$C_2H_5$ | 6-$C_2H_5$ | H | $CH_3$ | $CH_3$ | H | $CH_3$ | Cl(Br) |
| 2-$C_2H_5$ | 6-$C_2H_5$ | H | $CH_3$ | $CH_3$ | H | H | Cl(Br) |
| 2-$C_2H_5$ | 6-$C_2H_5$ | H | $CH_3$ | H | H | $CH_3$ | Cl(Br) |
| 2-$C_2H_5$ | 6-$C_2H_5$ | H | $CH_3$ | $CH_3$ | Cl | $CH_3$ | Cl(Br) |
| 2-$C_2H_5$ | 6-$C_2H_5$ | H | $CH_3$ | H | $OCH_3$ | H | Cl(Br) |
| 2-$CH_3$ | 3-$CH_3$ | H | $CH_3$ | H | H | H | Cl(Br) |
| 2-$CH_3$ | 3-$CH_3$ | H | $CH_3$ | H | Cl | H | Cl(Br) |
| 2-$CH_3$ | 3-$CH_3$ | H | $CH_3$ | $CH_3$ | H | $CH_3$ | Cl(Br) |
| 2-$CH_3$ | 3-$CH_3$ | H | $CH_3$ | $CH_3$ | H | H | Cl(Br) |
| 2-$CH_3$ | 3-$CH_3$ | H | $CH_3$ | H | H | $CH_3$ | Cl(Br) |
| 2-$CH_3$ | 3-$CH_3$ | H | $CH_3$ | $CH_3$ | Cl | $CH_3$ | Cl(Br) |
| 2-$CH_2$ | 3-$CH_3$ | H | $CH_3$ | H | $OCH_3$ | H | Cl(Br) |
| 2-$CH_3$ | 3-$CH_3$ | 4-$CH_3$ | $CH_3$ | H | H | H | Cl(Br) |
| 2-$CH_3$ | 3-$CH_3$ | 4-$CH_3$ | $CH_3$ | H | Cl | H | Cl(Br) |
| 2-$CH_3$ | 3-$CH_3$ | 4-$CH_3$ | $CH_3$ | $CH_3$ | H | $CH_3$ | Cl(Br) |
| 2-$CH_3$ | 3-$CH_3$ | 4-$CH_3$ | $CH_3$ | $CH_3$ | H | H | Cl(Br) |
| 2-$CH_3$ | 3-$CH_3$ | 4-$CH_3$ | $CH_3$ | H | H | $CH_3$ | Cl(Br) |
| 2-$CH_3$ | 3-$CH_3$ | 4-$CH_3$ | $CH_3$ | $CH_3$ | Cl | $CH_3$ | Cl(Br) |
| 2-$CH_3$ | 3-$CH_3$ | 4-$CH_3$ | $CH_3$ | H | $OCH_3$ | H | Cl(Br) |
| 2-$CH_3$ | 5-$CH_3$ | H | $CH_3$ | H | H | H | Cl(Br) |
| 2-$CH_3$ | 5-$CH_3$ | H | $CH_3$ | H | Cl | H | Cl(Br) |

Fortsetzung

| $X^1$ | $X^2$ | $X^3$ | R | $R^1$ | $R^2$ | $R^3$ | Hal |
|---|---|---|---|---|---|---|---|
| 2-$CH_3$ | 5-$CH_3$ | H | $CH_3$ | $CH_3$ | H | $CH_3$ | Cl(Br) |
| 2-$CH_3$ | 5-$CH_3$ | H | $CH_3$ | $CH_3$ | H | H | Cl(Br) |
| 2-$CH_3$ | 5-$CH_3$ | H | $CH_3$ | H | H | $CH_3$ | Cl(Br) |
| 2-$CH_3$ | 5-$CH_3$ | H | $CH_3$ | $CH_3$ | Cl | $CH_3$ | Cl(Br) |
| 2-$CH_3$ | 5-$CH_3$ | H | $CH_3$ | H | $OCH_3$ | H | Cl(Br) |
| 3-$CH_3$ | 5-$CH_3$ | H | $CH_3$ | H | H | H | Cl(Br) |
| 3-$CH_3$ | 5-$CH_3$ | H | $CH_3$ | H | Cl | H | Cl(Br) |
| 3-$CH_3$ | 5-$CH_3$ | H | $CH_3$ | $CH_3$ | H | $CH_3$ | Cl(Br) |
| 3-$CH_3$ | 5-$CH_3$ | H | $CH_3$ | $CH_3$ | H | H | Cl(Br) |
| 3-$CH_3$ | 5-$CH_3$ | H | $CH_3$ | H | H | $CH_3$ | Cl(Br) |
| 3-$CH_3$ | 5-$CH_3$ | H | $CH_3$ | $CH_3$ | Cl | $CH_3$ | Cl(Br) |
| 3-$CH_3$ | 5-$CH_3$ | H | $CH_3$ | H | $OCH_3$ | H | Cl(Br) |
| H | H | H | i-$C_3H_7$ | H | H | H | Cl(Br) |
| H | H | H | i-$C_3H_7$ | H | Cl | H | Cl(Br) |
| H | H | H | i-$C_3H_7$ | $CH_3$ | H | $CH_3$ | Cl(Br) |
| H | H | H | i-$C_3H_7$ | $CH_3$ | H | H | Cl(Br) |
| H | H | H | i-$C_3H_7$ | H | H | $CH_3$ | Cl(Br) |
| H | H | H | i-$C_3H_7$ | $CH_3$ | Cl | $CH_3$ | Cl(Br) |
| H | H | H | i-$C_3H_7$ | H | $OCH_3$ | H | Cl(Br) |
| 2-$CH_3$ | H | H | i-$C_3H_7$ | H | H | H | Cl(Br) |
| 2-$CH_3$ | H | H | i-$C_3H_7$ | H | Cl | H | Cl(Br) |
| 2-$CH_3$ | H | H | i-$C_3H_7$ | $CH_3$ | H | $CH_3$ | Cl(Br) |
| 2-$CH_3$ | H | H | i-$C_3H_7$ | $CH_3$ | H | H | Cl(Br) |
| 2-$CH_3$ | H | H | i-$C_3H_7$ | H | H | $CH_3$ | Cl(Br) |
| 2-$CH_3$ | H | H | i-$C_3H_7$ | $CH_3$ | Cl | $CH_3$ | Cl(Br) |
| 2-$CH_3$ | H | H | i-$C_3H_7$ | H | $OCH_3$ | H | Cl(Br) |
| 2-$C_2H_5$ | H | H | i-$C_3H_7$ | H | H | H | Cl(Br) |
| 2-$C_2H_5$ | H | H | i-$C_3H_7$ | H | Cl | H | Cl(Br) |
| 2-$C_2H_5$ | H | H | i-$C_3H_7$ | $CH_3$ | H | $CH_3$ | Cl(Br) |
| 2-$C_2H_5$ | H | H | i-$C_3H_7$ | $CH_3$ | H | H | Cl(Br) |
| 2-$C_2H_5$ | H | H | i-$C_3H_7$ | H | H | $CH_3$ | Cl(Br) |

Fortsetzung

| $X^1$ | $X^2$ | $X^3$ | R | $R^1$ | $R^2$ | $R^3$ | Hal |
|---|---|---|---|---|---|---|---|
| 2-$C_2H_5$ | H | H | i-$C_3H_7$ | $CH_3$ | Cl | $CH_3$ | Cl(Br) |
| 2-$C_2H_5$ | H | H | i-$C_3H_7$ | H | $OCH_3$ | H | Cl(Br) |
| 2-i-$C_3H_7$ | H | H | i-$C_3H_7$ | H | H | H | Cl(Br) |
| 2-i-$C_3H_7$ | H | H | i-$C_3H_7$ | H | Cl | H | Cl(Br) |
| 2-i-$C_3H_7$ | H | H | i-$C_3H_7$ | $CH_3$ | H | $CH_3$ | Cl(Br) |
| 2-i-$C_3H_7$ | H | H | i-$C_3H_7$ | $CH_3$ | H | H | Cl(Br) |
| 2-i-$C_3H_7$ | H | H | i-$C_3H_7$ | H | H | $CH_3$ | Cl(Br) |
| 2-i-$C_3H_7$ | H | H | i-$C_3H_7$ | $CH_3$ | Cl | $CH_3$ | Cl(Br) |
| 2-i-$C_3H_7$ | H | H | i-$C_3H_7$ | H | $OCH_3$ | H | Cl(Br) |
| 2-sek.-$C_4H_9$ | H | H | i-$C_3H_7$ | H | H | H | Cl(Br) |
| 2-sek.-$C_4H_9$ | H | H | i-$C_3H_7$ | H | Cl | H | Cl(Br) |
| 2-sek.-$C_4H_9$ | H | H | i-$C_3H_7$ | $CH_3$ | H | $CH_3$ | Cl(Br) |
| 2-sek.-$C_4H_9$ | H | H | i-$C_3H_7$ | $CH_3$ | H | H | Cl(Br) |
| 2-sek.-$C_4H_9$ | H | H | i-$C_3H_7$ | H | H | $CH_3$ | Cl(Br) |
| 2-sek.-$C_4H_9$ | H | H | i-$C_3H_7$ | $CH_3$ | Cl | $CH_3$ | Cl(Br) |
| 2-sek.-$C_4H_9$ | H | H | i-$C_3H_7$ | H | $OCH_3$ | H | Cl(Br) |
| 2-tert.-$C_4H_9$ | H | H | i-$C_3H_7$ | H | H | H | Cl(Br) |
| 2-tert.-$C_4H_9$ | H | H | i-$C_3H_7$ | H | Cl | H | Cl(Br) |
| 2-tert.-$C_4H_9$ | H | H | i-$C_3H_7$ | $CH_3$ | H | $CH_3$ | Cl(Br) |
| 2-tert.-$C_4H_9$ | H | H | i-$C_3H_7$ | $CH_3$ | H | H | Cl(Br) |
| 2-tert.-$C_4H_9$ | H | H | i-$C_3H_7$ | H | H | $CH_3$ | Cl(Br) |
| 2-tert.-$C_4H_9$ | H | H | i-$C_3H_7$ | $CH_3$ | Cl | $CH_3$ | Cl(Br) |
| 2-tert.-$C_4H_9$ | H | H | i-$C_3H_7$ | H | $OCH_3$ | H | Cl(Br) |
| 2-$CH_3$ | 6-$CH_3$ | H | i-$C_3H_7$ | H | H | H | Cl(Br) |
| 2-$CH_3$ | 6-$CH_3$ | H | i-$C_3H_7$ | H | Cl | H | Cl(Br) |
| 2-$CH_3$ | 6-$CH_3$ | H | i-$C_3H_7$ | $CH_3$ | H | $CH_3$ | Cl(Br) |
| 2-$CH_3$ | 6-$CH_3$ | H | i-$C_3H_7$ | $CH_3$ | H | H | Cl(Br) |
| 2-$CH_3$ | 6-$CH_3$ | H | i-$C_3H_7$ | H | H | $CH_3$ | Cl(Br) |
| 2-$CH_3$ | 6-$CH_3$ | H | i-$C_3H_7$ | $CH_3$ | Cl | $CH_3$ | Cl(Br) |
| 2-$CH_3$ | 6-$CH_3$ | H | i-$C_3H_7$ | H | $OCH_3$ | H | Cl(Br) |
| 2-$C_2H_5$ | 6-$CH_3$ | H | i-$C_3H_7$ | H | H | H | Cl(Br) |

Fortsetzung

| $X^1$ | $X^2$ | $X^3$ | R | $R^1$ | $R^2$ | $R^3$ | Hal |
|---|---|---|---|---|---|---|---|
| 2-$C_2H_5$ | 6-$CH_3$ | H | i-$C_3H_7$ | H | Cl | H | Cl(Br) |
| 2-$C_2H_5$ | 6-$CH_3$ | H | i-$C_3H_7$ | $CH_3$ | H | $CH_3$ | Cl(Br) |
| 2-$C_2H_5$ | 6-$CH_3$ | H | i-$C_3H_7$ | $CH_3$ | H | H | Cl(Br) |
| 2-$C_2H_5$ | 6-$CH_3$ | H | i-$C_3H_7$ | H | H | $CH_3$ | Cl(Br) |
| 2-$C_2H_5$ | 6-$CH_3$ | H | i-$C_3H_7$ | $CH_3$ | Cl | $CH_3$ | Cl(Br) |
| 2-$C_2H_5$ | 6-$CH_3$ | H | i-$C_3H_7$ | H | $OCH_3$ | H | Cl(Br) |
| 2-$C_2H_5$ | 6-$C_2H_5$ | H | i-$C_3H_7$ | H | H | H | Cl(Br) |
| 2-$C_2H_5$ | 6-$C_2H_5$ | H | i-$C_3H_7$ | H | Cl | H | Cl(Br) |
| 2-$C_2H_5$ | 6-$C_2H_5$ | H | i-$C_3H_7$ | $CH_3$ | H | $CH_3$ | Cl(Br) |
| 2-$C_2H_5$ | 6-$C_2H_5$ | H | i-$C_3H_7$ | $CH_3$ | H | H | Cl(Br) |
| 2-$C_2H_5$ | 6-$C_2H_5$ | H | i-$C_3H_7$ | H | H | $CH_3$ | Cl(Br) |
| 2-$C_2H_5$ | 6-$C_2H_5$ | H | i-$C_3H_7$ | $CH_3$ | Cl | $CH_3$ | Cl(Br) |
| 2-$C_2H_5$ | 6-$C_2H_5$ | H | i-$C_3H_7$ | H | $OCH_3$ | H | Cl(Br) |
| 2-$CH_3$ | 3-$CH_3$ | H | i-$C_3H_7$ | H | H | H | Cl(Br) |
| 2-$CH_3$ | 3-$CH_3$ | H | i-$C_3H_7$ | H | Cl | H | Cl(Br) |
| 2-$CH_3$ | 3-$CH_3$ | H | i-$C_3H_7$ | $CH_3$ | H | $CH_3$ | Cl(Br) |
| 2-$CH_3$ | 3-$CH_3$ | H | i-$C_3H_7$ | $CH_3$ | H | H | Cl(Br) |
| 2-$CH_3$ | 3-$CH_3$ | H | i-$C_3H_7$ | H | H | $CH_3$ | Cl(Br) |
| 2-$CH_3$ | 3-$CH_3$ | H | i-$C_3H_7$ | $CH_3$ | Cl | $CH_3$ | Cl(Br) |
| 2-$CH_3$ | 3-$CH_3$ | H | i-$C_3H_7$ | H | $OCH_3$ | H | Cl(Br) |
| 2-$CH_3$ | 3-$CH_3$ | 4-$CH_3$ | i-$C_3H_7$ | H | H | H | Cl(Br) |
| 2-$CH_3$ | 3-$CH_3$ | 4-$CH_3$ | i-$C_3H_7$ | H | Cl | H | Cl(Br) |
| 2-$CH_3$ | 3-$CH_3$ | 4-$CH_3$ | i-$C_3H_7$ | $CH_3$ | H | $CH_3$ | Cl(Br) |
| 2-$CH_3$ | 3-$CH_3$ | 4-$CH_3$ | i-$C_3H_7$ | $CH_3$ | H | H | Cl(Br) |
| 2-$CH_3$ | 3-$CH_3$ | 4-$CH_3$ | i-$C_3H_7$ | H | H | $CH_3$ | Cl(Br) |
| 2-$CH_3$ | 3-$CH_3$ | 4-$CH_3$ | i-$C_3H_7$ | $CH_3$ | Cl | $CH_3$ | Cl(Br) |
| 2-$CH_3$ | 3-$CH_3$ | 4-$CH_3$ | i-$C_3H_7$ | H | $OCH_3$ | H | Cl(Br) |
| 2-$CH_3$ | 5-$CH_3$ | H | i-$C_3H_7$ | H | H | H | Cl(Br) |
| 2-$CH_3$ | 5-$CH_3$ | H | i-$C_3H_7$ | H | Cl | H | Cl(Br) |
| 2-$CH_3$ | 5-$CH_3$ | H | i-$C_3H_7$ | $CH_3$ | H | $CH_3$ | Cl(Br) |
| 2-$CH_3$ | 5-$CH_3$ | H | i-$C_3H_7$ | $CH_3$ | H | H | Cl(Br) |
| 2-$CH_3$ | 5-$CH_3$ | H | i-$C_3H_7$ | H | H | $CH_3$ | Cl(Br) |

Fortsetzung

| $X^1$ | $X^2$ | $X^3$ | R | $R^1$ | $R^2$ | $R^3$ | Hal |
|---|---|---|---|---|---|---|---|
| 2-$CH_3$ | 5-$CH_3$ | H | i-$C_3H_7$ | $CH_3$ | Cl | $CH_3$ | Cl(Br) |
| 2-$CH_3$ | 5-$CH_3$ | H | i-$C_3H_7$ | H | $OCH_3$ | H | Cl(Br) |
| 2-$CH_3$ | H | H | $C_2H_5$ | H | H | H | Cl(Br) |
| 2-$CH_3$ | 6-$CH_3$ | H | $C_2H_5$ | H | H | H | Cl(Br) |
| 2-$CH_3$ | 6-$C_2H_5$ | H | $C_2H_5$ | H | H | H | Cl(Br) |
| 2-$C_2H_5$ | 6-$C_2H_5$ | H | $C_2H_5$ | H | H | H | Cl(Br) |
| 2-$CH_3$ | H | H | $C_2H_5$ | H | Cl | H | Cl(Br) |
| 2-$CH_3$ | 6-$CH_3$ | H | $C_2H_5$ | H | Cl | H | Cl(Br) |
| 2-$CH_3$ | 6-$C_2H_5$ | H | $C_2H_5$ | H | Cl | H | Cl(Br) |
| 2-$C_2H_5$ | 6-$C_2H_5$ | H | $C_2H_5$ | H | Cl | H | Cl(Br) |
| 2-$CH_3$ | H | H | $C_2H_5$ | $CH_3$ | H | $CH_3$ | Cl(Br) |
| 2-$CH_3$ | 6-$CH_3$ | H | $C_2H_5$ | $CH_3$ | H | $CH_3$ | Cl(Br) |
| 2-$CH_3$ | 6-$C_2H_5$ | H | $C_2H_5$ | $CH_3$ | H | $CH_3$ | Cl(Br) |
| 2-$C_2H_5$ | 6-$C_2H_5$ | H | $C_2H_5$ | $CH_3$ | H | $CH_3$ | Cl(Br) |
| 2-$CH_3$ | H | H | $C_2H_5$ | $CH_3$ | Cl | $CH_3$ | Cl(Br) |
| 2-$CH_3$ | 6-$CH_3$ | H | $C_2H_5$ | $CH_3$ | Cl | $CH_3$ | Cl(Br) |
| 2-$CH_3$ | 6-$C_2H_5$ | H | $C_2H_5$ | $CH_3$ | Cl | $CH_3$ | Cl(Br) |
| 2-$C_2H_5$ | 6-$C_2H_5$ | H | $C_2H_5$ | $CH_3$ | Cl | $CH_3$ | Cl(Br) |
| 2-$CH_3$ | H | H | n-$C_3H_7$ | H | H | H | Cl(Br) |
| 2-$CH_3$ | 6-$CH_3$ | H | n-$C_3H_7$ | H | H | H | Cl(Br) |
| 2-$CH_3$ | 6-$C_2H_5$ | H | n-$C_3H_7$ | H | H | H | Cl(Br) |
| 2-$C_2H_5$ | 6-$C_2H_5$ | H | n-$C_3H_7$ | H | H | H | Cl(Br) |
| 2-$CH_3$ | H | H | n-$C_3H_7$ | H | Cl | H | Cl(Br) |
| 2-$CH_3$ | 6-$CH_3$ | H | n-$C_3H_7$ | H | Cl | H | Cl(Br) |
| 2-$CH_3$ | 6-$C_2H_5$ | H | n-$C_3H_7$ | H | Cl | H | Cl(Br) |
| 2-$C_2H_5$ | 6-$C_2H_5$ | H | n-$C_3H_7$ | H | Cl | H | Cl(Br) |
| 2-$CH_3$ | H | H | n-$C_3H_7$ | $CH_3$ | H | $CH_3$ | Cl(Br) |
| 2-$CH_3$ | 6-$CH_3$ | H | n-$C_3H_7$ | $CH_3$ | H | $CH_3$ | Cl(Br) |
| 2-$CH_3$ | 6-$C_2H_5$ | H | n-$C_3H_7$ | $CH_3$ | H | $CH_3$ | Cl(Br) |
| 2-$C_2H_5$ | 6-$C_2H_5$ | H | n-$C_3H_7$ | $CH_3$ | H | $CH_3$ | Cl(Br) |
| 2-$CH_3$ | H | H | n-$C_3H_7$ | $CH_3$ | Cl | $CH_3$ | Cl(Br) |

Fortsetzung

| $X^1$ | $X^2$ | $X^3$ | R | $R^1$ | $R^2$ | $R^3$ | Hal |
|---|---|---|---|---|---|---|---|
| $2\text{-}CH_3$ | $6\text{-}CH_3$ | H | $n\text{-}C_3H_7$ | $CH_3$ | Cl | $CH_3$ | Cl(Br) |
| $2\text{-}CH_3$ | $6\text{-}C_2H_5$ | H | $n\text{-}C_3H_7$ | $CH_3$ | Cl | $CH_3$ | Cl(Br) |
| $2\text{-}C_2H_5$ | $6\text{-}C_2H_5$ | H | $n\text{-}C_3H_7$ | $CH_3$ | Cl | $CH_3$ | Cl(Br) |

Verwendet man beispielsweise 2-Ethyl-6-methyl-chloracetanilid und N-(1-Chlorethyl)-pyrazol-hydrochlorid als Ausgangsstoffe, so kann der Reaktionsablauf des erfindungsgemäßen Verfahrens durch das folgende Formelschema wiedergegeben werden:

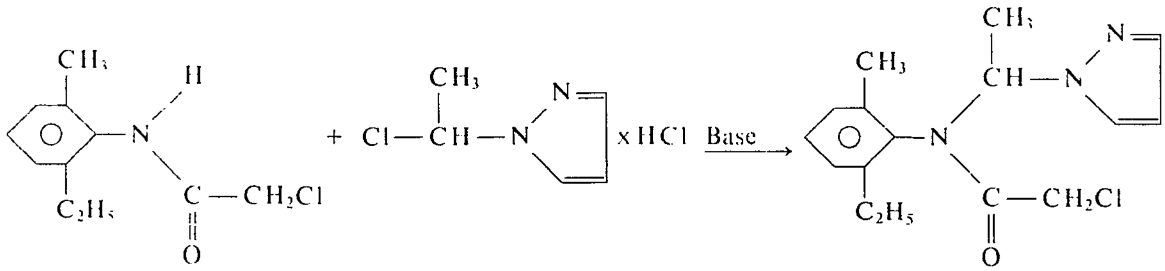


Die bei der Durchführung des erfindungsgemäßen Verfahrens als Ausgangsstoffe benötigten Halogenacetanilide sind durch die Formel (II) allgemein definiert. In dieser Formel stehen Hal, $X^1$, $X^2$ und $X^3$ für diejenigen Reste, die bereits im Zusammenhang mit der Beschreibung der erfindungsgemäßen Stoffe der Formel (I) für diese Reste genannt wurden.

Die Halogenacetanilide der Formel (II) sind allgemein bekannt bzw. können sie in allgemein bekannter Art und Weise erhalten werden, indem man entsprechende Aniline mit einem Halogenacethalogenid oder Halogenacetanhydrid in Gegenwart eines inerten organischen Lösungsmittels, wie beispielsweise Toluol oder Dimethylformamid, gegebenenfalls in Gegenwart eines Säurebindemittels, wie z. B. Kaliumcarbonat, bei Temperaturen zwischen 20 und 100°C umsetzt (vgl. auch die Herstellungsbeispiele).

Als Beispiele seien genannt:

Chlor-(brom)acetanilid 2-Methyl-chlor(brom)-acetanilid;
2-Ethyl-chlor(brom)acetanilid; 2-Isopropyl-chlor(brom)acetanilid;
2-sek.-Butyl-chlor(brom)acetanilid; 2-tert.-Butylchlor(brom)acetanilid;
2,6-Dimethyl-chlor(brom)acetanilid; 2,3-Dimethyl-chlor(brom)acetanilid;
2,5-Dimethyl-chlor(brom)acetanilid; 3,5-Dimethyl-chlor(brom)acetanilid;
2,6-Diethyl-chlor(brom)acetanilid; 2-Ethyl-6-methyl-chlor(brom)acetanilid;
2,3,4-Trimethyl-chlor(brom)acetanilid; 2,4,6-Trimethyl-chlor(brom)acetanilid;
2,4,5-Trimethyl-chlor(brom)acetanilid; 2-Ethyl-4,6-dimethyl-chlor(brom)acetanilid;
2,6-Diethyl-4-methyl-chlor(brom)acetanilid;
2,6-Diisopropyl-4-methyl-chlor(brom)acetanilid;
2,3,5-Trimethyl-chlor(brom)acetanilid.

Die außerdem für die erfindungsgemäße Umsetzung als Ausgangsstoffe zu verwendenden Pyrazolyl-Derivate sind durch die Formel (III) allgemein definiert. In dieser Formel stehen R, $R^1$, $R^2$ und $R^3$ für diejenigen Reste, die bereits im Zusammenhang mit der Beschreibung der erfindungsgemäßen Stoffe der Formel (I) für diese Reste genannt wurden. Y steht vorzugsweise für die Halogene Chlor und Brom sowie für den Mesylat- und Tosylat-Rest.

Die Pyrazolyl-Derivate der Formel (III) sind noch nicht bekannt. Man erhält sie, wenn man bekannte Pyrazole der Formel

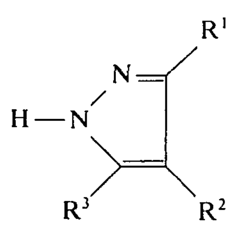
(IV)

in welcher
$R^1$, $R^2$ und $R^3$ die oben angegebene Bedeutung haben,

mit einem bekannten Aldehyd der Formel

$$O{=}CH{-}R \qquad (V)$$

in welcher
R die oben angegebene Bedeutung hat,
gegebenenfalls in Gegenwart eines inerten organischen Lösungsmittels, wie beispielsweise Methylenchlorid, bei Temperaturen zwischen −70 und +50°C, vorzugsweise bei −20 bis +30°C umsetzt und die dabei entstehenden neuen Verbindungen der Formel

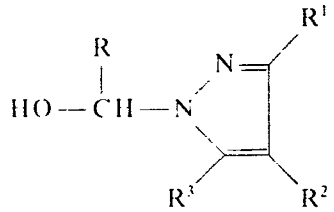
(VI)

in welcher
R, $R^1$, $R^2$ und $R^3$ die oben angegebene Bedeutung haben,
direkt oder gegebenenfalls nach Isolierung mit einem Halogenierungsmittel, wie beispielsweise Thionylchlorid oder Phosphortribromid, oder mit einem Sulfonylierungsmittel, wie Mesylchlorid oder Tosylchlorid, gegebenenfalls in Gegenwart eines inerten organischen Lösungsmittels, wie z. B. Methylenchlorid, bei Temperaturen zwischen −70°C und +50°C, vorzugsweise bei −20°C bis +30°C umsetzt. Dabei fallen die Pyrazolyl-Derivate der Formel (III) in Form von Halogenwasserstoff-Salzen an, je nach verwendetem Halgenierungsmittel bzw. Sulfonylierungsmittel. Die Pyrazolyl-Derivate können anschließend in üblicher Weise in Freiheit gesetzt werden, sie können aber auch in Form ihrer anfallenden Halogenwasserstoff-Salze direkt weiter umgesetzt werden (vgl. auch die Herstellungsbeispiele).

Die Verbindungen der Formel (VI) stehen im chemischen Gleichgewicht mit ihren Ausgangsstoffen und sind deshalb nur in Einzelfällen isolierbar.

Als Beispiele für die Pyrazolyl-Derivate der Formel (III) seien genannt:

1-(1′-Brom(chlor)ethyl)-pyrazol, 1-(1′-Brom(chlor)ethyl)-4-chlorpyrazol,
1-(1′-Brom(chlor)ethyl)-2-methyl-pyrazol,
1-(1′-Brom(chlor)ethyl)-5-methyl-pyrazol,
1-(1′-Brom(chlor)ethyl)-3,5-dimethyl-pyrazol,
1-(1′-Brom(chlor)ethyl)-4-chlor-3,5-dimethyl-pyrazol,
1-(1′-Brom(chlor)ethyl)-4-methoxy-pyrazol, 1-(1′-Brom(chlor)propyl)-pyrazol,
1-(1′-Brom(chlor)propyl)-4-chlor-pyrazol
1-(1′-Brom(chlor)propyl)-3-methyl-pyrazol,
1-(1′-Brom(chlor)propyl)-5-methyl-pyrazol, 1-(1′-Brom(chlor)propyl)-3,5-dimethyl-pyrazol,
1-(1′-Brom(chlor)propyl)-4-chlor-3,5-dimethyl-pyrazol,
1-(1′-Brom(chlor)propyl)-4-methoxy-pyrazol, 1-(1′-Brom(chlor)butyl)-pyrazol,
1-(1′-Brom(chlor)butyl)-4-chlorpyrazol, 1-(1′-Brom(chlor)butyl)-2-methyl-pyrazol,
1-(1′-Brom(chlor)butyl)-5-methyl-pyrazol, 1-(1′-Brom(chlor)butyl)-3,5-dimethyl-pyrazol,
1-(1′-Brom(chlor)butyl)-4-chlor-3,5-dimethyl-pyrazol,
1-(1′-Brom(chlor)butyl)-4-methoxy-pyrazol, 1-(1′-Brom(chlor)-2′-methyl-propyl)-pyrazol,
1-(1′-Brom(chlor)-2′-methyl-propyl)-4-chlor-pyrazol,
1-(1′-Brom(chlor)-2′-methyl-propyl)-3-methyl-pyrazol,
1-(1′-Brom(chlor)-2′-methyl-propyl)-5-methyl-pyrazol,
1-(1′-Brom(chlor)-2′-methyl-propyl)-3,5-dimethyl-pyrazol,
1-(1′-Brom(chlor)-2′-methyl-propyl)-4-chlor-3,5-dimethyl-pyrazol,
1-(1′-Brom(chlor)-2′-methyl-propyl)-4-methoxy-pyrazol,
1-(1′-Brom(chlor)pentyl)-pyrazol, 1-(1′-Brom(chlor)pentyl)-4-chlor-pyrazol,
1-(1′-Brom(chlor)pentyl)-3-methyl-pyrazol, 1-(1′-Brom(chlor)pentyl)-5-methylpyrazol,
1-(1′-Brom(chlor)pentyl)-3,5-dimethyl-pyrazol,
1-(1′-Brom(chlor)pentyl)-4-chlor-3,5-dimethyl-pyrazol,
1-(1′-Brom(chlor)pentyl-4-methoxy-pyrazol.

Zur Herstellung von Säureadditionssalzen der Verbindungen der Formel (I) kommen alle Säuren infrage, die zu physiologisch verträglichen Salzen führen. Hierzu gehören vorzugsweise die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure,

Bernsteinsäure, Fumarsäure, Weinsäure, Zitronensäure, Salizylsäure, Sorbinsäure, Milchsäure, sowie Sulfonsäuren, wie z. B. p-Toluolsulfonsäure und 1,5-Naphthalindisulfonsäure.

Die Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien. Als Anionen der Salze kommen solche in Betracht, die sich von solchen Säuren ableiten, die zu physiologisch verträglichen Salzen führen. Hierzu gehören vorzugsweise die Halogenwasserstoffsäuren, wie z. B. die Chlorwasserstoffsäure und die Bromwasserstoffsäure, ferner Phosphorsäure, Salpetersäure und Schwefelsäure.

Die Metallsalzkomplexe der Verbindungen der Formel (I) können in einfacher Weise nach üblichen Verfahren erhalten werden, so z. B. durch Lösen des Metallsalzes in Alkohol, z. B. Äthanol, und Hinzufügen zur Verbindung der Formel (I). Man kann Metallsalzkomplexe in bekannter Weise, z. B. durch Abfiltrieren, isolieren und gegebenenfalls durch Umkristallisieren reinigen.

Als Lösungsmittel kommen für die erfindungsgemäße Umsetzung alle inerten, mit Wasser nicht mischbaren, organischen Lösungsmittel infrage. Hierzu gehören vorzugsweise Ether, wie Diethylether; aromatische Kohlenwasserstoffe, wie Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Die erfindungsgemäße Umsetzung wird in Gegenwart eines Säurebindemittels durchgeführt. Als solche konnen alle üblichen Säurebindemittel verwendet werden. Hierzu gehören vorzugsweise anorganische Basen, wie beispielsweise Alkalihydroxide und Alkalicarbonate.

Die Reaktionstemperaturen können bei der Durchführung des erfindungsgemäßen Verfahrens in einem größeren Bereich variiert werden. Im allgemeinen arbeitet man zwischen −70° C und +100° C, vorzugsweise zwischen −20° C und +80° C.

Bei der Durchführung des erfindungsgemäßen Verfahrens setzt man vorzugsweise auf 1 Mol Halogenacetanilid der Formel (II) 0,5 bis 2,0 Mol Pyrazol-Derivat der Formel (III) sowie 1 bis 10 Mol Säurebinder ein. Die Isolierung der Verbindung der Formel (I) erfolgt in üblicher Weise.

In einer bevozugten Ausführungsform wird die erfindungsgemäße Umsetzung in einem Zweiphasensystem, wie beispielsweise wäßrige Natron- oder Kalilauge/Toluol oder Methylenchlorid, gegebenenfalls unter Zusatz von 0,01—1 Mol eines Phasen-Transfer-Katalysators, wie beispielsweise Ammonium- oder Phosphoniumverbindungen, beispielsweise sei Benzyl-dodecyl-dimethyl-ammoniumchlorid (Zephirol) genannt, durchgeführt.

Nach einer Variante des erfindungsgemäßen Verfahrens ist es auch möglich, Aniline der Formel

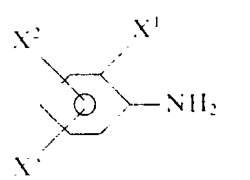

(VII)

in welcher

$X^1$, $X^2$ und $X^3$ die oben angegebene Bedeutung haben,

entsprechend der Durchführung der erfindungsgemäßen Verfahrens zunächst mit Pyrazol-Derivaten der Formel (III) umzusetzen, wobei als Säurebinder auch ein Überschuß an Anilin der Formel (VII) verwendet werden kann, und anschließend die so erhaltenen substituierten sekundären Aniline der Formel

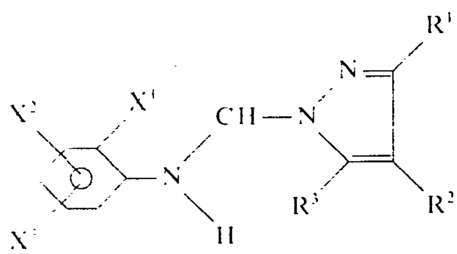

(VIII)

in welcher

R, $R^1$, $R^2$, $R^3$, $X^1$, $X^2$ und $X^3$ die oben angegebene Bedeutung haben,

mit einem Halogenacethalogenid oder Halogenacetanhydrid in Gegenwart eines inerten organischen

Lösungsmittels, wie beispielsweise Toluol oder Dimethylformamid, gegebenenfalls in Gegenwart eines Säurebindemittels, wie z. B. Kaliumcarbonat, bei Temperaturen zwischen 20°C und 100°C umzusetzen.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden:

Dikotyle Unkräuter der Gattungen:
Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:
Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen:
Enchinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Spenoclea, Dactyloctenium, Agrostis, Alopecurus, Apera.

Monokotyle Kulturen der Gattungen:
Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen insbesondere neben einer sehr guten Wirkung gegen grasartige Unkräuter auch eine gute herbizide Wirkung bei greitblättrigen Unkräutern. Ein Einsatz der erfindungsgemäßen Wirkstoffe zur selektiven Unkrautbekämpfung ist möglich, vorzugsweise in Mais, Erdnüssen, Rüben, Sojabohnen, Baumwolle, Reis und Getreidearten.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsionskonzentrate, Wirkstoff-imprägnierte Natur- und synthetische Stoffe, Feinverkapselungen in polymeren Stoffen.

Diese Formulierungen werden in bekannter Weise hergestellt, z. B. durch Vermischen der Wirkstoffe mit Streckmitteln also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von schaumerzeugenden Mitteln. Im Falle der Benutzung von schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Diemethylsulfoxid, sowie Wasser.

Als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und

synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,1 und 10 kg Wirkstoff pro ha, vorzugsweise zwischen 0,1 und 5 kg/ha.

Die erfindungsgemäßen Wirkstoffe zeigen bei Nachauflaufanwendung auch wachstumsregulierende Eigenschaften und eignen sich als Wachstumshemmer.

Wirkung und Einsatzmöglichkeiten der erfindungsgemäßen Stoffe zur Umkrautbekämpfung gehen aus dem Beispiel A hervor.

In dem folgenden Beispiel werden die nachstehend genannten Stoffe eingesetzt:

Wirkstoffliste

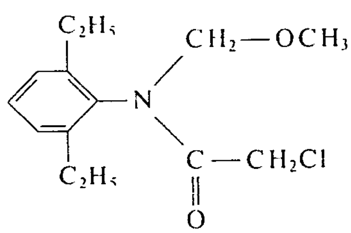

(A)

(bekannt aus US-PS 34 42 945)

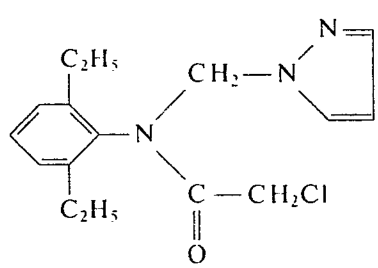

(B)

(bekannt aus FR-PS 23 68 476 und DE-OS 27 04 281)

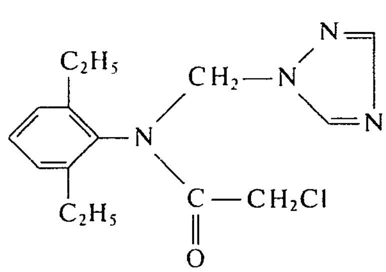

(C)

(bekannt aus FR-PS 23 68 476 und DE-OS 27 04 281)

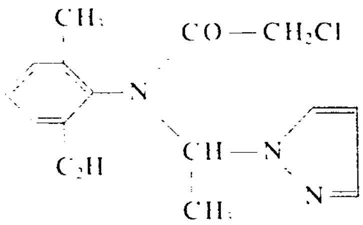

(1)

(erfindungsgemäß)

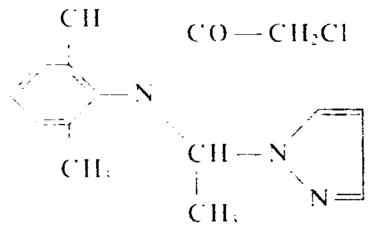

(2)

(erfindungsgemäß)

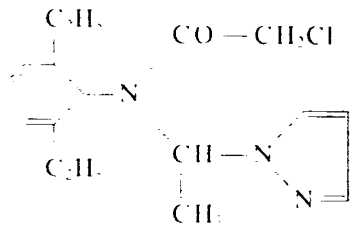

(3)

(erfindungsgemäß)

Beispiel A

Pre-emergence-Test

Lösungsmittel: 5 Gewichtsteile Aceton
Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Wirkstoffe, Aufwandmengen und Resultate werden ermittelt.

Die erfindungsgemäßen Wirkstoffe (2) und (3) zeigen in diesem Test eine bessere selektive Wirksamkeit als die aus dem Stand der Technik bekannte Substanz (A).

Tabelle A

Pre-emergence-Test

| Wirkstoff | Wirkstoff-aufwand kg/ha | Echino-chloa | Setaria | Sinapis | Matri caria | Galinsoga | Stellaria | Lolium | Avena fatua | Baum-wolle | Mais | Rüben |
|---|---|---|---|---|---|---|---|---|---|---|---|---|
| (A) | 5 | 100 | 100 | 20 | 90 | 100 | 90 | 90 | 80 | 20 | 40 | 60 |
| (bekannt) | 2,5 | 90 | 100 | 0 | 90 | 100 | 90 | 90 | 80 | 0 | 0 | 50 |
| (3) | 5 | 100 | 90 | 20 | 80 | 100 | 80 | 80 | 60 | 40 | 60 | 0 |
| | 2,5 | 80 | 80 | 0 | 80 | 100 | 60 | 60 | 60 | 0 | 40 | 0 |
| (2) | 5 | 100 | 100 | 80 | 80 | 100 | 100 | 100 | 80 | 40 | 60 | 60 |
| | 2,5 | 100 | 100 | 0 | 80 | 100 | 100 | 100 | 80 | 0 | 60 | 40 |

Tabelle B

Pre-emergence-Test

| Wirkstoff | kg/ha | % Wirkung Sinapis | Galinsoga | Stellaria | Lolium |
|---|---|---|---|---|---|
| (2) | 2,5 | 0 | 100 | 100 | 100 |
| (B) | 2,5 | 40 | 100 | 90 | 90 |

Tabelle C

Pre-emergence-Test

| Wirkstoff | Wirkstoffaufwandmenge kg/ha | Setaria | Matricaria | Galinsoga | Lolium | Hirse | Weizen | Baumwolle | Mais | Rüben |
|---|---|---|---|---|---|---|---|---|---|---|
| (C) | 2,5 | 90 | 70 | 80 | 60 | 90 | 0 | 0 | 0 | 0 |
| | 1,25 | 90 | 60 | 80 | 0 | 80 | 0 | 0 | 0 | 0 |
| (2) | 2,5 | 100 | 80 | 100 | 80 | 100 | 60 | 20 | 0 | 40 |
| | 1,25 | 100 | 80 | 90 | 70 | 80 | 20 | 0 | 0 | 40 |
| (1) | 2,5 | 100 | 90 | 90 | 80 | 100 | 0 | 0 | 60 | 0 |
| | 1,25 | 100 | 80 | 90 | 70 | 80 | 0 | 0 | 60 | 0 |

## Herstellungsbeispiele

### Beispiel 1

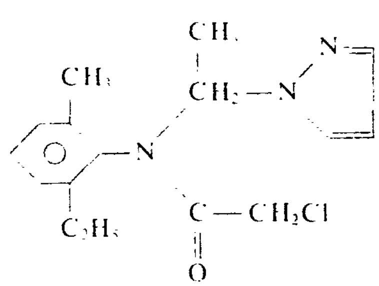

42,3 g (0,2 Mol) 2-Ethyl-6-methyl-chloracetanilid und 36,8 g (0,22 Mol) N-(1-Chlorethyl)-pyrazol-hydrochlorid werden in 200 ml Methylenchlorid gelöst. Nach Zugabe von 0,5 ml Zephirol (50%ige wäßrige Lösung von Benzyl-dodecyl-dimethylammoniumchlorid) tropft man unter starkem Rühren eine Lösung von 80 g (2 Mol) Natriumhydroxid in 80 ml Wasser ein, wobei sich das Reaktionsgemisch bis zum Rückfluß erhitzt. Man rührt ca. 3 Stunden weiter, bis das Reaktionsgemisch auf Raumtemperatur abgekühlt ist. Die organische Phase wird abgetrennt, mehrfach mit Wasser neutral gewaschen, über Natriumsulfat getrocknet und im Vakuum eingeengt. Man erhält ein farbloses Öl als Rückstand, das nach einiger Zeit durchkristallisiert. Ausbeute 29 g (47,5% der Theorie) 2-Ethyl-6-methyl-N-(pyrazol-1-yl-eth-1-yl)-chloracetanilid in Form weißer Kristalle vom Schmelzpunkt 74° C. Die Substanz kann durch mehrfaches Umkristallisieren aus Diethylether gereinigt werden und hat dann einen Schmelzpunkt von 84° C.

Herstellung der Vorprodukte

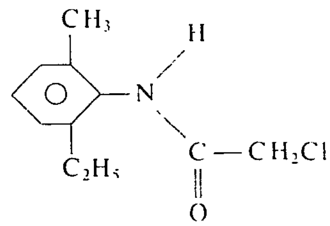

(II-1)

135,2 g (1 Mol) 2-Ethyl-6-methyl-anilin in 1000 ml Toluol werden mit 152 g (1,1 Mol) Kaliumcarbonat versetzt. Dazu werden unter Rühren 113 g (1 Mol) Chloressigsäurechlorid getropft. Nach Abklingen der exothermen Reaktion läßt man 2 Stunden unter Rückfluß nachrühren. Anschließend wird das Reaktionsgemisch filtriert und das Filtrat im Vakuum auf 500 ml eingeengt. Die dabei entstehenden Kristalle werden abgesaugt und mit Petrolether gewaschen. Man erhält 202,9 g (96,2% der Theorie) 2-Ethyl-6-methyl-chloracetanilid in Form weißer Kristalle vom Schmelzpunkt 120° C.

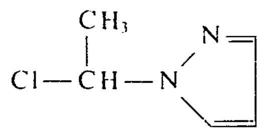 X HCl

(III-1)
(VI-1)

Zu 340 g (5 Mol) Pyrazol in 1200 ml Methylenchlorid werden bei 0 bis 5° C innerhalb einer Stunde 250 g (5,7 Mol) Acetaldehyd getropft. Man läßt ca. 1 Stunde bei 0° C nachrühren. Das dabei entstehende N-(1-Hydroxyethyl)-pyrazol wird nicht isoliert, sondern die Reaktionslösung wird direkt bei 0 bis 5° C innerhalb einer Stunde zu 1250 g (10,5 Mol) Thionylchlorid getropft. Man läßt 1 Stunde bei 20° C nachrühren und engt dann bei 40° C im Vakuum ein. Nach Zugabe von 300 ml Methylenchlorid wird erneut eingeengt. Der Rückstand wird im Vakuum destilliert. Man erhält 620,3 g (75% der Theorie) N-(1-Chlorethyl)-pyrazol-hydrochlorid vom Siedepunkt 55° C/18 mm.

In entsprechender Weise werden diejenigen Verbindungen erhalten, die in der Tabelle 2 formelmäßig aufgeführt sind.

Tabelle 2

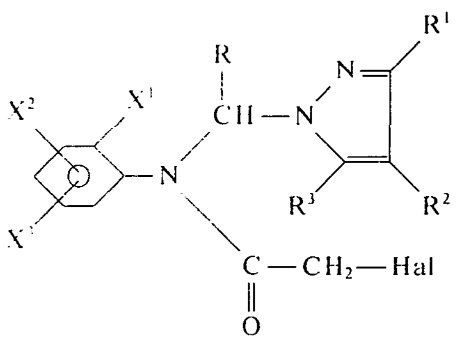

(I)

| Bsp. Nr. | $X^1$ | $X^2$ | $X^3$ | R | $R^1$ | $R^2$ | $R^3$ | Hal | Schmelzpunkt (°C) |
|---|---|---|---|---|---|---|---|---|---|
| 2 | $CH_3$ | 6-$CH_3$ | H | $CH_3$ | H | H | H | Cl | 90 |
| 3 | $C_2H_5$ | 6-$C_2H_5$ | H | $CH_3$ | H | H | H | Cl | 80 |
| 4 | $CH_3$ | 6-$C_2H_5$ | H | $CH_3$ | H | H | H | Cl | 95 (x HCl) |

Nach bekanntem Verfahren werden die in der nachstehenden Tabelle 3 formelmäßig aufgeführten Ausgangsprodukte der Formel (II) erhalten.

Tabelle 3

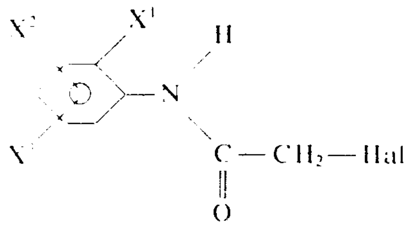

(II)

| Beispiel Nr. | $X^1$ | $X^2$ | $X^3$ | Hal | Schmelzpunkt (°C) |
|---|---|---|---|---|---|
| II-2 | $CH_3$ | 6-$CH_3$ | H | Cl | 148 |
| II-3 | $C_2H_5$ | 6-$C_2H_5$ | H | Cl | 133 |
| II-4 | i-$C_3H_7$ | H | H | Cl | 79 |
| II-5 | tert.-$C_4H_9$ | H | H | Cl | 96 |
| II-6 | $C_2H_5$ | H | H | Cl | 103 |
| II-7 | $CH_3$ | H | H | Cl | 109 |
| II-8 | $CH_3$ | 3-$CH_3$ | H | Cl | 135 |
| II-9 | $CH_3$ | 5-$CH_3$ | H | Cl | 154 |
| II-10 | $CH_3$ | 4-$CH_3$ | 6-$CH_3$ | Cl | 177 |
| II-11 | $C_2H_5$ | 4-$CH_3$ | 6-$CH_3$ | Cl | 134 |
| II-12 | sek.-$C_4H_9$ | H | H | Cl | Öl |
| II-13 | H | H | H | Cl | 132 |

Nach dem in der Anmeldung beschriebenen Verfahren werden die in der nachstehenden Tabelle 4 formelmäßig aufgeführten Ausgangsprodukte der Formel (III) erhalten.

Tabelle 4

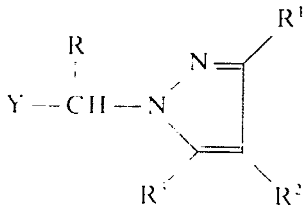

(III)

| Beispiel Nr. | R | $R^1$ | $R^2$ | $R^3$ | Y | Schmelzpunkt (°C) Siedepunkt (°C) |
|---|---|---|---|---|---|---|
| III 2 | $-i-C_3H_7$ | H | H | H | Cl | Fp: 114°C (x HCl) |
| III 3 | $-CCl_3$ | H | H | H | Cl | Fp: 95°C (x HCl) |
| III 4 | $-CH_3$ | $CH_3$ | Cl | $CH_3$ | Cl | Öl (x HCl) |
| III 5 | $-CH_3$ | H | Cl | H | Cl | Sdp: $60-62/_{20\,mb}$ (x HCl) |
| III 6 | $-C_2H_5$ | H | H | H | Cl | Öl (x HCl) |
| III 7 | $-n-C_3H_7$ | H | H | H | Cl | Öl (x HCl) |
| III 8 | $-CH_3$ | H | H | H | Br | Sdp: $60-65/_{20\,mb}$ |
| III 9 | $-CH_3$ | H | H | H | Cl | Sdp: $50-55/_{18\,mb}$ |

Nach dem in der Anmeldung beschriebenen Verfahren werden die in der nachstehenden Tabelle 5 formelmäßig aufgeführten Zwischenprodukte der Formel (VI) erhalten.

Tabelle 5

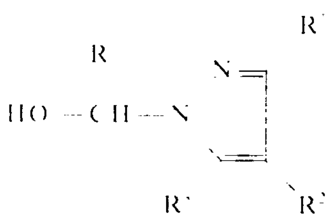

(IV)

| Beispiel Nr. | R | $R^1$ | $R^2$ | $R^3$ | Physikalische Konstante |
|---|---|---|---|---|---|
| VI-2 | $i-C_3H_7$ | H | H | H | nicht isoliert |
| VI-3 | $-CCl_3$ | H | H | H | Fp: 109°C |
| VI-4 | $-CH_3$ | $CH_3$ | Cl | $CH_3$ | Fp: 39−44°C |

**Patentansprüche für die Vertragsstaaten: BE, DE, FR, IT, NL, SE, CH und GB**

1. Substituierte N-Pyrazolylmethyl-halogenacetanilide der Formel

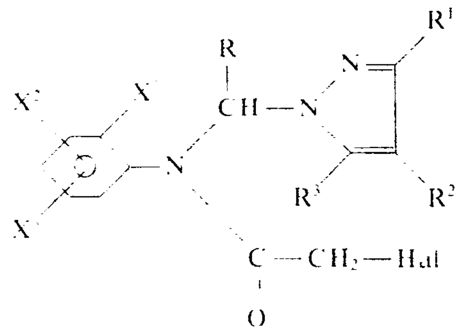

(I)

in welcher

Hal für Fluor, Chlor oder Brom steht,
R für Alkyl mit 1 bis 8 Kohlenstoffatomen, Cycloalkyl mit 3 bis 6 Kohlenstoffatomen, Halogenalkyl mit bis zu 2 Kohlenstoffatomen und bis zu 3 Halogenatomen, Alkoxyalkyl mit 1 bis 4 Kohlenstoffatomen im Alkylteil und 1 bis 4 Kohlenstoffatomen im Alkoxyteil, für Alkenyl mit 2 bis 4 Kohlenstoffatomen, Alkinyl mit 2 bis 4 Kohlenstoffatomen oder für gegebenenfalls durch Halogen, Alkyl mit 1 bis 4 Kohlenstoffatomen, Halogenalkyl mit bis zu 2 Kohlenstoffatomen und bis zu 3 Halogenatomen, Alkoxy mit bis zu 2 Kohlenstoffatomen, Alkylthio mit bis zu 2 Kohlenstoffatomen, Cyano und/oder Nitro substituiertes Phenyl steht,
$R^1$, $R^2$ und $R^3$ gleich oder verschieden sind und für Wasserstoff, Alkyl mit 1 bis 4 Kohlenstoffatomen, Halogen oder Alkoxy mit 1 bis 4 Kohlenstoffatomen stehen und
$X^1$, $X^2$ und $X^3$ gleich oder verschieden sind und für Wasserstoff oder Alkyl mit 1 bis 4 Kohlenstoffatomen stehen,

sowie deren Säureadditions-Salze und Metallsalz-Komplexe.

2. Verfahren zur Herstellung von substituierten N-pyrazolylmethyl-halogenacetaniliden der Formel (I) gemäß Anspruch 1 sowie deren Säureadditions-Salzen und Metallsalz-Komplexen, dadurch gekennzeichnet, daß man Halogenacetanilide der Formel

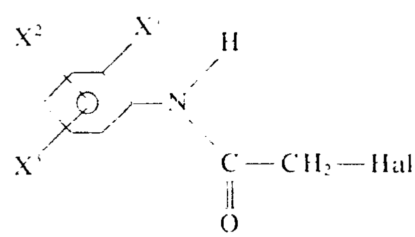

(II)

in welcher
Hal, $X^1$, $X^2$ und $X^3$ die oben angegebene Bedeutung haben,
mit Pyrazolyl-Derivaten der Formel

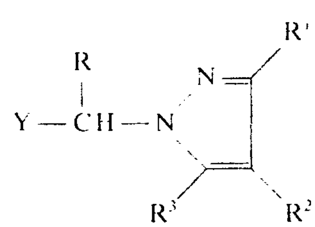

(III)

in welcher
R, $R^1$, $R^2$ und $R^3$ die oben angegebene Bedeutung haben und
Y für Halogen, den Mesyl- oder Tosyl-Rest steht,
in Gegenwart eines Säurebinders und gegebenenfalls in Gegenwart eines organischen Lösungsmittels umsetzt und gegebenenfalls anschließend eine Säure oder ein Metallsalz, addiert, wobei die Pyrazol-Derivate der Formel (III) vorzugsweise in Form von Halogenwasserstoffsalzen eingesetzt werden.

3. Herbizide Mittel, gekennzeichnet durch einen Gehalt an mindestens einem substituierten N-Pyrazolylmethylhalogenacetanilid der Formel (I) gemäß Anspruch 1.

4. Verwendung von substituierten N-Pyrazolylmethylhalogenacetaniliden der Formel (I) gemäß Anspruch 1 zur Bekämpfung von Unkräutern.

**Patentansprüche für den Vertragsstaat: AT**

1. Verfahren zur Herstellung von substituierten N-Pyrazolylmethyl-halogenacetaniliden der Formel

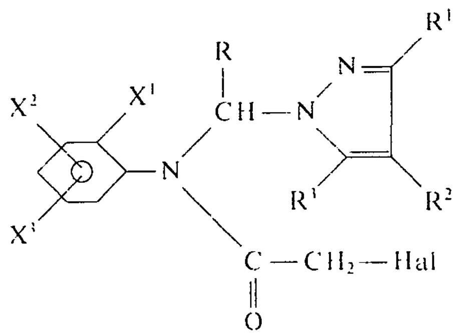

(I)

in welcher

Hal für Fluor, Chlor oder Brom steht,

R für Alkyl mit 1 bis 8 Kohlenstoffatomen, Cycloalkyl mit 3 bis 6 Kohlenstoffatomen, Halogenalkyl mit bis zu 2 Kohlenstoffatomen und bis zu 3 Halogenatomen, Alkoxyalkyl mit 1 bis 4 Kohlenstoffatomen im Alkylteil und 1 bis 4 Kohlenstoffatomen im Alkoxyteil, für Alkenyl mit 2 bis 4 Kohlenstoffatomen, Alkinyl mit 2 bis 4 Kohlenstoffatomen oder für gegebenenfalls durch Halogen, Alkyl mit 1 bis 4 Kohlenstoffatomen, Halogenalkyl mit bis zu 2 Kohlenstoffatomen und bis zu 3 Halogenatomen, Alkoxy mit bis zu 2 Kohlenstoffatomen, Alkylthio mit bis zu 2 Kohlenstoffatomen, Cyano und/oder Nitro substituiertes Phenyl steht,

$R^1$, $R^2$ und $R^3$ gleich oder verschieden sind und für Wasserstoff, Alkyl mit 1 bis 4 Kohlenstoffatomen, Halogen oder Alkoxy mit 1 bis 4 Kohlenstoffatomen stehen und

$X^1$, $X^2$ und $X^3$ gleich oder verschieden sind und für Wasserstoff oder Alkyl mit 1 bis 4 Kohlenstoffatomen stehen,

sowie deren Säureadditions-Salze und Metallsalz-Komplexe, dadurch gekennzeichnet, daß man Halogenacetanilide der Formel

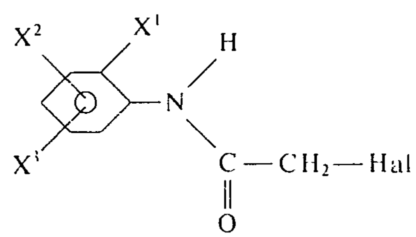

(II)

in welcher

Hal, $X^1$, $X^2$ und $X^3$ die oben angegebene Bedeutung haben,

mit Pyrazolyl-Derivaten der Formel

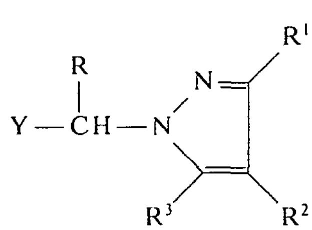

(III)

in welcher

R, $R^1$, $R^2$ und $R^3$ die oben angegebene Bedeutung haben und

Y für Halogen, den Mesyl- oder Tosyl-Rest steht,

in Gegenwart eines Säurebinders und gegebenenfalls in Gegenwart eines organischen Lösungsmittels umsetzt und gegebenenfalls anschließend eine Säure oder ein Metallsalz, addiert, wobei die Pyrazol-Derivate der Formel (III) vorzugsweise in Form von Halogenwasserstoffsalzen eingesetzt werden.

2. Herbizide Mittel, gekennzeichnet durch einen Gehalt an mindestens einem substituierten N-Pyrazolylmethylhalogenacetanilid der Formel (I) gemäß Anspruch 1.

3. Verwendung von substituierten N-Pyrazolylmethylhalogenacetaniliden der Formel (I) gemäß Anspruch 1 zur Bekämpfung von Unkräutern.

**Claims BE, CH, DE, FR, GB, JT, NL, SE**

1. Substituted N-pyrazolylmethyl-halogenoacetanilides of the formula

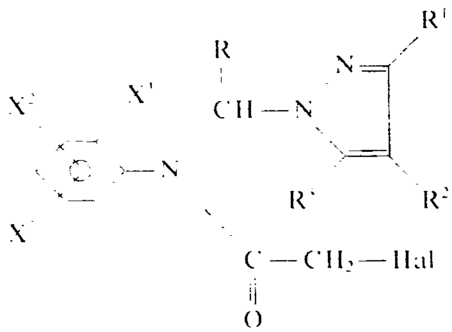

(I)

in which

Hal represents fluorine, chlorine or bromine,

R represents alkyl with 1 to 8 carbon atoms, cycloalkyl with 3 to 6 carbon atoms, halogenoalkyl with up to 2 carbon atoms and up to 3 halogen atoms, alkoxyalkyl with 1 to 4 carbon atoms in the alkyl part and 1 to 4 carbon atoms in the alkoxy part, alkenyl with 2 to 4 carbon atoms, alkinyl with 2 to 4 carbon atoms or phenyl optionally substituted by halogen, alkyl with 1 to 4 carbon atoms, halogenoalkyl with up to 2 carbon atoms and up to 3 halogen atoms, alkoxy with up to 2 carbon atoms, alkylthio with up to 2 carbon atoms, cyano and/or nitro,

$R^1$, $R^2$ and $R^3$ are identical or different and represent hydrogen, alkyl with 1 to 4 carbon atoms, halogen or alkoxy with 1 to 4 carbon atoms and

$X^1$, $X^2$ and $X^3$ are identical or different and represent hydrogen or alkyl with 1 to 4 carbon atoms,

and acid addtion salts and metal salt complexes thereof.

2. Process for the preparation of substituted N-pyrazolylmethyl-halogenoacetanilides of the formula (I) according to Claim 1 and acid addition salts and metal salt complexes thereof, characterised in that halogenoacetanilides of the formula

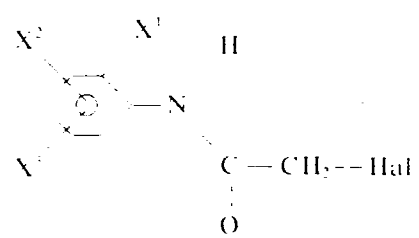

(II)

in which

Hal, $X^1$, $X^2$ and $X^3$ have the meaning indicated above, are reacted with pyrazolyl derivatives of the formula

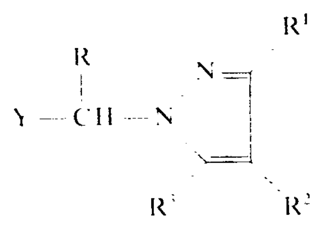

(III)

in which

R, $R^1$, $R^2$ and $R^3$ have the meaning indicated above and

Y represents halogen or the mesylate or tosylate radical,

in the presence of an acid-binding agent and if appropriate in the presence of an organic solvent, and an acid or a metal salt is then optionally added on, the pyrazole derivatives of the formula (III) preferably being employed in the form of hydrogen halide salts.

3. Herbicidal agents, characterised in that they contain at least one substituted N-pyrazolylmethyl-halogenoacetanilide of the formula (I) according to Claim 1.

4. Use of substituted N-pyrazolylmethyl-halogenoacetanilides of the formula (I) according to Claim 1, for combating weeds.

**Claims for Austria**

1. Process for the preparation of substituted N-pyrazolylmethyl-halogenoacetanilides of the formula

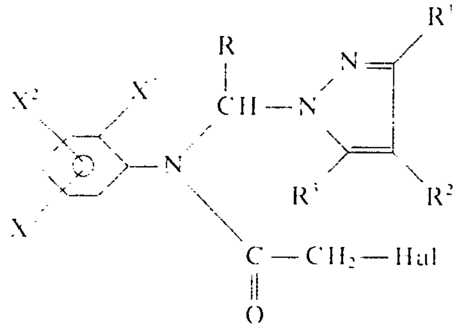

(I)

in which

Hal represents fluorine, chlorine or bromine,

R represents alkyl with 1 to 8 carbon atoms, cycloalkyl with 3 to 6 carbon atoms, halogenoalkyl with up to 2 carbon atoms and up to 3 halogen atoms, alkoxyalkyl with 1 to 4 carbon atoms in the alkyl part and 1 to 4 carbon atoms in the alkoxy part, alkenyl with 2 to 4 carbon atoms, alkinyl with 2 to 4 carbon atoms or phenyl optionally substituted by halogen, alkyl with 1 to 4 carbon atoms, halogenoalkyl with up to 2 carbon atoms and up to 3 halogen atoms, alkoxy with up to 2 carbon atoms, alkylthio with up to 2 carbon atoms, cyano and/or nitro,

$R^1$, $R^2$ and $R^3$ are identical or different and represent hydrogen, alkyl with 1 to 4 carbon atoms, halogen or alkoxy with 1 to 4 carbon atoms and

$X^1$, $X^2$ ans $X^3$ are identical or different and represent hydrogen or alkyl with 1 to 4 carbon atoms,

and acid addition salts and metal salt complexes thereof, characterised in that halogenoacetanilides of the formula

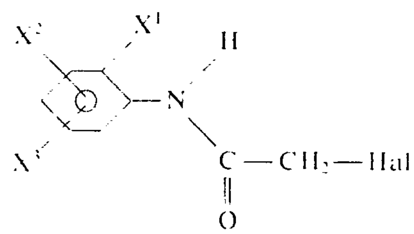

(II)

in which

Hal, $X^1$, $X^2$ and $X^3$ have the meaning indicated above, are reacted with pyrazolyl derivatives of the formula

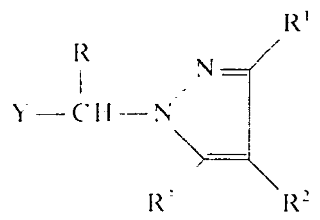

(III)

in which

R, $R^1$, $R^2$ and $R^3$ have the meaning indicated above and

Y represents halogen or the mesylate or tosylate radical,

in the presence of an acid-binding agent and if appropriate in the presence of an organic solvent, and an acid or a metal salt is then optionally added on, the pyrazole diervatives of the formula (III) preferably being employed in the form of hydrogen halide salts.

2. Herbicidal agents, characterised in that they contain at least one substituted N-pyrazolylmethyl-halogenoactanilide of the formula (I) according to Claim 1.

3. Use of substituted N-pyrazolylmethyl-halogenoacetanilides of the formula (I) according to Claim 1, for combating weeds.

**Revendications BE, CH, DE, FR, GB, IT, NL, SE**

1. N-pyrazolylrnéthyl-halogénacétanilides substitués de formule:

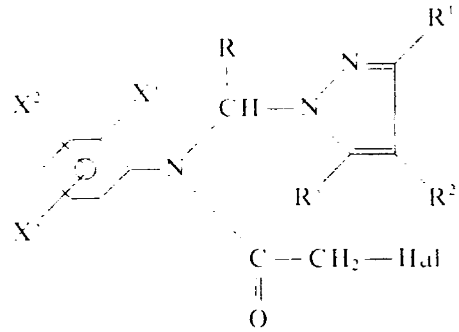

(I)

dans laquelle

Hal représente un atome de fluor, un atome de chlore ou un atome de brome,

R représente un groupe alkyle contenant 1 à 8 atomes de carbone, un groupe cycloalkyle contenant 3 à 6 atomes de carbone, un groupe halogénalkyle contenant jusqu'à 2 atomes de carbone et jusqu'à 3 atomes d'halogènes, un groupe alcoxyalkyle contenant 1 à 4 atomes de carbone dans la fraction alkyle et 1 à 4 atomes de carbone dans la fraction alcoxy, un groupe alcényle contenant 2 à 4 atomes de carbone, un groupe alcinyle contenant 2 à 4 atomes de carbone ou un groupe phényle éventuellement substitué par un atome d'halogène, par un groupe alkyle contenant 1 à 4 atomes de carbone, par un groupe halogénalkyle contenant jusqu'à 2 atomes de carbone et jusqu'à 3 atomes d'halogènes, par un groupe alcoxy contenant jusqu'à 2 atomes de carbone, par un groupe alkylthio contenant jusqu'à 2 atomes de carbone, par un groupe cyano et/ou par un groupe nitro,

$R^1$, $R^2$ et $R^3$ sont identiques ou différents et représentent chacun un atome d'hydrogène, un groupe alkyle contenant 1 à 4 atomes de carbone, un atome d'halogène ou un groupe alcoxy contenant 1 à 4 atomes de carbone, et

$X^1$, $X^2$ et $X^3$ sont identiques ou différents et représentent chacun un atome d'hydrogène ou un groupe alkyle contenant 1 à 4 atomes de carbone,

ainsi que leurs sels d'addition d'acide et leurs complexes de sels métalliques.

2. Procédé de préparation de N-pyrazolylmethyl-halogénacétanilides substitués de formule (I) suivant la revendication 1, ainsi que de leurs sels d'addition d'acide et de leurs complexes de sels métalliques, caractérisé en ce qu'on fait réagir des halogénacétanilides de formule:

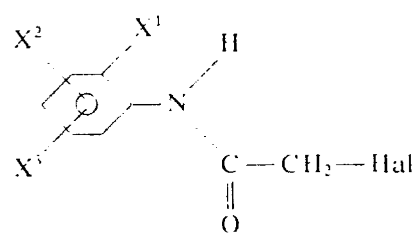

(II)

dans laquelle
Hal, $X^1$, $X^2$ et $X^3$ ont les significations indiquées cidessus.

avec des dérivés pyrazolyle de formule:

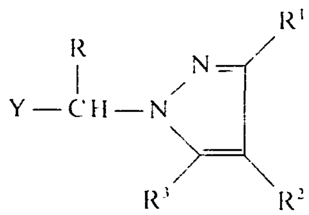

(III)

dans laquelle
R, $R^1$, $R^2$ et $R^3$ ont les significations indiquées cidessus, et
Y représente un atome d'halogène, le groupe mésyle ou le groupe tosyle,
en présence d'un fixateur d'acide et éventuellement en présence d'un solvant organique, puis on ajoute éventuellement un acide ou un sel métallique, les dérivés pyrazole de formule (III) étant utilisés, de préférence, sous forme de sels d'hydracides halogénés.

3. Agents herbicides, caractérisés en ce qu'ils contiennent au moins un N-pyrazolylméthyl-halogénacétanilide substitué de formule (I) suivant la revendication 1.

4. Utilisation de N-pyrazolylméthyl-halogénacétanilides substitués de formule (I) suivant la revendication 1 en vue de combattre les mauvaises herbes.

**Revendications pour Autriche**

1. Procédé de préparation de N-pyrazolyl-methyl-halogen-acétanilides substitués de formule:

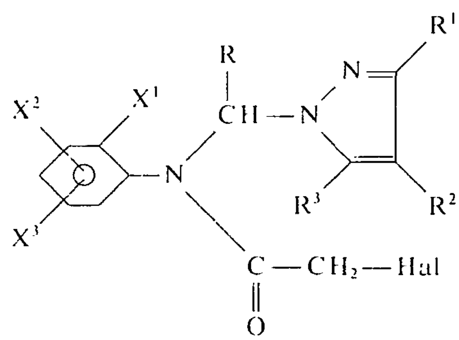

(I)

dans laquelle

Hal représente un atome de fluor, un atome de chlore ou un atome de brome,
R représente un groupe alkyle contenant 1 à 8 atomes de carbone, un groupe cycloalkyle contenant 3 à 6 atomes de carbone, un groupe halogénalkyle contenant jusqu'à 2 atomes de carbone et jusqu'à 3 atomes d'halogènes, un groupe alcoxyalkyle contenant 1 à 4 atomes de carbone dans la fraction alkyle et 1 à 4 atomes de carbone dans la fraction alcoxy, un groupe alcényle contenant 2 à 4 atomes de carbone, un groupe alcinyle contenant 2 à 4 atomes de carbone ou un groupe phényle éventuellement substitué par un atome d'halogène, par un groupe alkyle contenant 1 à 4 atomes de carbone, par un groupe halogénalkyle contenant jusqu'à 2 atomes de carbone et jusqu'à 3 atomes d'halogènes, par un groupe alcoxy contenant jusqu'à 2 atomes de carbone, par un groupe alkylthio contenant jusqu'à 2 atomes de carbone, par un groupe cyano et/ou par un groupe nitro,
$R^1$, $R^2$ et $R^3$ sont identiques ou différents et représentent chacun un atome d'hydrogène, un groupe alkyle contenant 1 à 4 atomes de carbone, un atome d'halogène ou un groupe alcoxy contenant 1 à 4 atomes de carbone, et
$X^1$, $X^2$ et $X^3$ sont identiques ou différents et représentent chacun un atome d'hydrogène ou un groupe alkyle contenant 1 à 4 atomes de carbone,

ainsi que leurs sels d'addition d'acide et leurs complexes de sels métalliques, caractérisé en ce qu'on fait réagir des halogénacétanilides de formule:

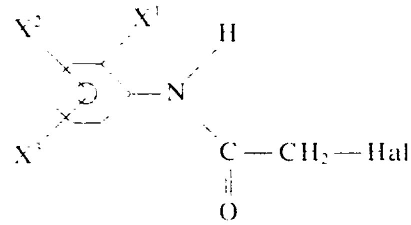

(II)

dans laquelle
Hal, $X^1$, $X^2$ et $X^3$ ont les significations indiquées cidessus,
avec des dérivés pyrazolyle de formule:

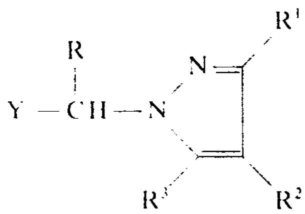

(III)

dans laquelle
R, $R^1$, $R^2$ et $R^3$ ont les significations indiquées cidessus, et
Y représente un atome d'halogène, le groupe mésyle ou le groupe tosyle,
en présence d'un fixateur d'acide et éventuellement en présence d'un solvant organique, puis on ajoute éventuellement un acide ou un sel métallique, les dérivés pyrazole de formule (III) étant utilisés, de préférence, sous forme de sels d'hydracides halogénés.

2. Agents herbicides, caractérisés en ce qu'ils contiennent au moins un N-pyrazolylméthyl.

3. Utilisation de N-pyrazolylméthyl-halogénacétanilides substitués de formule (I) suivant la revendication 1 en vue de combattre les mauvaises herbes.